# EUROPEAN PATENT APPLICATION

(11) **EP 2 153 834 A2**
(43) Date of publication of application: **17.02.2010**
(21) Application number: 09167343.4
(22) Date of filing: 06.08.2009
(51) Int. Cl.: A61K 31/55, A61K 9/20

(54) **Extended release pharmaceutical compositions comprising quetiapine salts**

(30) Priority: 07.08.2008 EP 08014092
(71) Applicant: Farmaprojects, S.A., 08902 L'Hospitalet de Llobregat (Barcelona) (ES)
(72) Inventor: Gual-Pujol, Francisco, 08902, L'Hospitalet de Llobregat (ES); Amela-Navarro, Joaquín, 08902, L'Hospitalet de Llobregat (ES)

(57) **Abstract**

An extended release multiparticulate system comprising quetiapine or a pharmaceutical acceptable salt, pharmaceutical acceptable excipients and at least one hydrophobic release controlling agent, wherein the hydrophobic release controlling agent ranges from 1 % to 25 % w/w, preferably from 5 % to 20 %, more preferably from 5% to 10%.

## Description

### FIELD OF THE INVENTION

The present invention relates to extended release pharmaceutical compositions comprising quetiapine or a pharmaceutically acceptable salt thereof and at least one hydrophobic release controlling agent and to methods for their manufacture.

### BACKGROUND OF THE INVENTION

Quetiapine acts as a 5HT2 and D2 receptor antagonist and is marketed as a hemifumarate, having the formula:

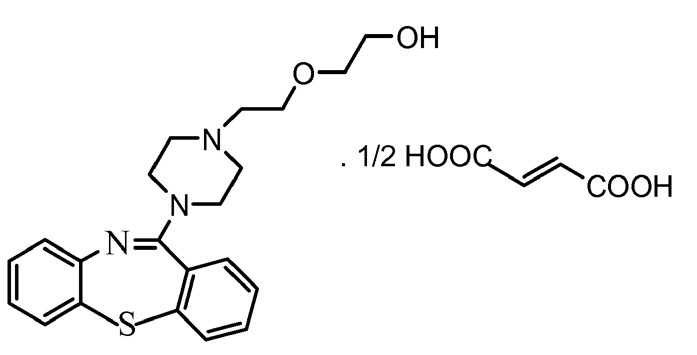

The preparation, physical properties and pharmacological properties of quetiapine, and its pharmaceutically acceptable salts are disclosed in EP 240228 A1 and EP 282236 A1 and in U.S. Patent No. 4,879,288.

Quetiapine and its pharmaceutically acceptable salts exhibit antidopaminergic activity and are used as an antipsychotic agent (for example, for the management of the manifestations of psychotic disorders) or as a treatment for hyperactivity. It shows a substantial reduction in the potential to cause side effects, such as acute dystonia, acute dyskinesia, pseudo-Parkinsonism and tardive dyskinesia, which may result from the use of other antipsychotics.

Quetiapine hemifumarate is soluble in water in acidic pHs (such as that of the stomach), so that it tends to generate an extended release product which is susceptible to a phenomenon known as dose dumping. Dose dumping is defined as a sudden release of the drug which may give rise to drug plasma levels higher than the minimal toxic dose, with the consequent adverse effects. Minimising the risk of dose-dumping is essential. However the present inventors have surprisingly discovered an extended release multiparticulate system which overcomes these problems.

Due to the rapid metabolism of quetiapine salts, immediate release tablets typically have to be administered two or more times a day. Such multiple daily administrations may result in poor patient compliance due to the number of tablets that have to be taken daily. Accordingly, it would be advantageous to provide a pharmaceutical composition which would deliver quetiapine in an extended release manner. Desirably this extended release form should provide a more uniform rate of release over an extended period of time, thus resulting in therapeutic blood (plasma) level of the active ingredient without the need for frequent administration of the medicament.

EP907364 A1 discloses extended release matrix compositions comprising a gelling agent, all the examples include hydroxypropyl methylcellulose (HPMC). It is well known that HPMC is frequently used in extended release formulations (Feely LC, Davis SS. Pharm Res. 1989 Apr;6(4):274-8). However, the presence of HPMC, due to its hydrophilicity, makes it more difficult the water granulation techniques (Int J. Pharm. 2006 Apr 26; 313(1-2):57-65).

WO 2005/041935 A1 discloses a solid dosage form comprising a matrix, wherein the matrix comprises: a therapeutically effective amount of quetiapine or a pharmaceutically acceptable salt thereof; and a wax material. Suitable waxes include beeswax, glycowax, castor wax, carnauba wax and like substances. The use of waxes requires melting and cooling of the wax to elaborate the dosage form and makes the manufacture more time consuming.

Providing an alternative to extended release matrix tablets or film-coated tablets with extended release coatings would be advantageous as it would avoid the risk of dose dumping. For example, the use film-coated tablets with extended release coatings often involves considerable risks. If the coating is damaged, the tablets may release a multiple dose of the individual drug in a very short time and cause harmful side effects by exceeding the toxic concentration of the drug (dose dumping).

### SUMMARY OF THE INVENTION

One problem to be solved by the present invention is to provide alternative, efficient, safe, cost-effective and stable pharmaceutical compositions comprising quetiapine hemifumarate. Additionally, the present compositions overcome, or at least alleviate, one or more of the above described difficulties and further maintain the advantageous property of enabling the active ingredient to be administered less frequently, e.g, once a day, while achieving blood (plasma) levels similar to those attained by administering immediate release doses of the medicament more frequently, e.g. two or more times daily. Moreover, another aim of the present invention is to simplify the clinical evaluation of such new quetiapine pharmaceutical composition. The number of bioequivalence studies needed with multiple doses is greatly reduced, since the delivery properties are intrinsically linked to the units (pellets, granules, mini-tablets, etc), and not the whole dosage form (tablet, capsule, etc.). Therefore the present inventors tried to find dosage forms in which individual smaller units are contained in one larger unit (multiparticulate system), from which they are released shortly after swallowing.

The first aspect of the present invention is an extended release multiparticulate system comprising quetiapine or a pharmaceutical acceptable salt, pharmaceutical acceptable excipients and at least one hydrophobic release controlling agent, wherein the hydrophobic release controlling agent ranges from 1 % to 25 % w/w, preferably 5 % - 20 %, more preferably 5 % - 10%.

A novel, suitable, stable, extended release pharmaceutical composition, which does not contain gelling agents or waxes as release controlling agents has been invented. It consists of a multiparticulate system which provides an alternative to extended release matrix tablets reducing the risk of dose dumping. On the one hand, this multiparticulate system comprises individual small units which release the active ingredient in a controlled manner. On the other hand, hydrophobic release controlling agents have not shown a tendency to produce dose dumping as compared with gelling agents.

The second aspect of the invention is a tablet or capsule comprising an extended release system according to the first aspect of the invention. Once the individual small units are prepared, they may be included in hard gelatine capsules or may be compressed to form tablets.

The third aspect of the invention is a method for the manufacture of a tablet according to the second aspect of the invention comprising at least the steps of:
a) Granulating a mixture of quetiapine hemifumarate and at least one pharmaceutical acceptable excipient with at least one hydrophobic release controlling agent,
b) Optionally, spheronizing the mixture to form pellets,
c) Drying the granulate or pellets,
d) Optionally, coating the dried product with at least one hydrophobic release controlling agent,
e) If needed, mixing the product obtained in the granulation or spheronization step with pharmaceutical acceptable excipients to obtain a compression mixture,
f) Compressing the previous mixture to obtain tablets, and optionally, applying a coating,

The granulation may be performed using the hydrophobic release controlling agent dispersed in an organic or aqueous granulation solution or added to the mixture of the active substance and other pharmaceutical acceptable excipients to be granulated with a solvent. The hydrophobic release controlling agent may be previously adsorbed into an adsorbing agent such as colloidal silica. In addition to this, the hydrophobic release controlling agent may be also added as a coating around the granule.

The tablet may be coated with one or more coatings well known in the art, such a non-functional film coating. However, when the multiparticulate system is based on pellets and they are compressed the tablet surface appears irregular and a thicker coating is necessary. The present inventors have discovered the use of sugar-free suspensions, for example with sorbitol, for coating extended release quetiapine tablets without modifying the release profile. This kind of coating based on polyols provides a good alternative to sugar coatings and makes the tablets suitable for use with diabetic patients.
Moreover, the present inventors have found that even when the extended release granulate or pellets are coated with other hydrophobic release controlling agents the batch to batch reproducibility is maintained.

The fourth aspect of the present invention is a method for the manufacture of a tablet according to the second aspect of the invention comprising at least the steps of:
a) Providing quetiapine hemifumarate,
b) Optionally, mixing it with at least one pharmaceutically acceptable excipient, and or with at least one hydrophobic release controlling agent until a homogenous mixture is formed,
c) Subjecting quetiapine hemifumarate or the mixture to compression,
d) Milling, grinding or sieving the compressed mixture,
e) Optionally, adding additional excipients and mixing the combination,
f) Compressing the pre-compressed product to form a tablet, and optionally coating.

The fifth aspect of the present invention is a method for the manufacture of a tablet according to the second aspect of the invention comprising at least the steps of:
a) Providing quetiapine hemifumarate,
b) Optionally, mixing it with at least one pharmaceutically acceptable excipient and or with at least one hydrophobic release controlling agent until a homogenous mixture is formed,
c) Subjecting quetiapine hemifumarate or the mixture to compression to form a tablet,
d) Optionally, coating.

The sixth aspect of the present invention is a method for the manufacture of a tablet according to the second aspect of the invention comprising at least the steps of:
a) Coating inert cores with a coating organic solution which comprises quetiapine hemifumarate and a hydrophobic release controlling agent,
b) If needed, mixing the coated cores with further pharmaceutical acceptable excipients to obtain a compression mixture,
c) Compressing the coated cores or the previous mixture to obtain tablets, and optionally, applying a coating.

Surprisingly it has also been possible to develop an extended release multiparticulate system, based on inert cores coated with a hydrophobic agent and quetiapine hemifumarate, which releases the active ingredient in a controlled manner. Since it is highly unlikely that the film coating of all these small units is damaged at the same time, such dosage forms are much safer and preferable to plain, film-coated, single units.

### Definitions

The present invention dicloses, but is not limited to quetiapine fumarate (2-[2-(4-dibenzo[b,f][1,4]thiazepin-11-yl-1-piperazinyl)ethoxy]-ethanol fumarate (2:1)) compositions. Extended release compositions according to the present invention may contain any quetiapine pharmaceutically acceptable salt.

Extended release is used to describe a formulation that does not release the active drug substance immediately after oral administration and that also enables a reduction in dosage frequency. The present extended release multiparticulate system can be based, but not limited, on beads, spheroids, microspheres, seeds, pellets, granules, minitablets or microtablets, and other multiparticulate systems in order to obtain a desired extended release of quetiapine hemifumarate. The dosage form of this multiparticulate system may be presented in tablets, capsules or other suitable unit dosage forms and preferably releases the active ingredient in a controlled manner over a period of up to about 6 hours or longer.

A hydrophobic release controlling agent is understood as being a molecule (known as a hydrophobe) that is repelled from a mass of water, but not necessarily lipophilic, such as silicones. For this reason, the term hydrophobic release controlling agent as used herein means a substance which does not form a gel when in contact with water. The present inventors do not include wax materials amongst the lipophilic substances, and use oily substances which have a melting point lower than 30°C. The suitable oily substances include organic oils, mineral oils and fatty acids. Those agents not considered as lipophilic are copolymers (derived from two (or more) monomeric species such as silicones, poly (lactic-co-glycolic acid) derivates, acrylic and methacrylic acid esters or polyvinyl alcohol-polyvinyl acetates.

Organic oils are produced by plants, animals and other organisms through organic processes, and these oils are remarkable in their diversity. Normally, oils are liquid at room temperature. Although thought of as esters of glycerin and a varying blend of fatty acids, these oils contain free fatty acids and also diglycerides. Mineral oil or liquid petrolatum is a by-product of the distillation of petroleum. It is transparent, colourless oil composed mainly of alkanes (typically 15 to 40 carbons) and cyclic paraffins, related to white petrolatum. These are classified as mineral oils as they do not have an organic origin on human timescales, and are instead derived from underground geologic locations, ranging from rocks, to underground traps, to sands.

Fatty acids are aliphatic monocarboxylic acids, derived from, or contained in esterified form in an animal or vegetable fat, oil or wax. Natural fatty acids commonly have a chain of 4 to 28 carbons (usually unbranched and even numbered), which may be saturated or unsaturated. The present invention uses liquid fatty acids at room temperature.

Carrier matrix is used to describe extended release microparticles based on a mixture of pharmaceutical acceptable excipients wherein the active substance and the hydrophobic release controlling agent are homogenously dispersed.

Sugar free inert cores are inert, non-hygroscopic, highly spherical starter cores made of polyols. These cores may be layered and coated and used in controlled release formulations. These inert cores have high mechanical strength, low attrition, and high density and well defined particle size distribution.

### DETAILED DESCRIPTION OF PARTICULAR EMBODIMENTS

In one embodiment of the first aspect of the invention, the system comprises at least one hydrophobic release controlling agent selected from oils, fatty acids, salts of fatty acids, and copolymers, polyvinyl acetate and mixtures thereof. These agents provide a release mechanism based on diffusion and erosion.

In another embodiment of the first aspect of the invention, the oil is selected from organic oils or mineral oils and mixtures thereof.

The present inventors have used oily substances (melting point lower than 30 ° C) as release controlling agents. Although there are frequent draw-backs as regards scale-up with the use of these substances, e.g. technological problems during compression, such as sticking, the present inventors have managed to overcome these problems. Moreover, using these oily substances instead of waxes makes the manufacturing process easier because two steps can be avoided; it is not necessary either to melt the substance before processing or to cool it once it is in the mixture.

In spite of the low melting point of these substances (lower than 30 °C) and the tendency to produce manufacturing problems such as sticking, the present inventors have managed to use them as hydrophobic release agents in queatipine multiparticulate systems without technological problems. It has been possible to combine them with suitable proportions of other pharmaceutical acceptable excipients and to employ commonly used methods of manufacture.

In yet another embodiment of the first aspect of the invention, the organic oil is selected from lindseed oil, tung oil, otica oil, soybean oil, cotton seed oil, castor oil, corn oil, olive oil, rapeseed oil, sunflower oil, fish oil, fish liver oil, oleic acid and mixtures thereof.

In yet another embodiment of the first aspect of the invention, the mineral oil is selected from of petroleum, refined paraffin oil and mixtures thereof.

In yet another embodiment of the first aspect of the invention, the fatty acid is selected from myristoleic acid, oleic acid, linoleic acid, linolenic acid, butanoic acid, hexanoic acid, caprylic acid, capric acid, palmitoleic acid, eicosapentaenoic acid, docosahexanoic acid.

In yet another embodiment of the first aspect of the invention, the salt of fatty acid is selected from magnesium stearate and calcium stearate.

In another embodiment of the first aspect of the invention, the copolymer is selected from poly (lactic-co-glycolic acid), siloxane, acrylic and methacrylic acid esters or polyvinyl alcohol-polyvinylacetate and mixtures thereof.

The use of copolymers of acrylic and methacrylic acid esters as hydrophofobic agents yields a considerable extended release effect in the first hours in the dissolution profile tests, which correlate with an improved therapeutic activity and which is particularly advantageous. In particular, this effect is seen especially with the use of Eudragit FS 30D.

Oils, fatty acids, magnesium or calcium stearate and the copolymers such as poly (lactic-co-glycolic acid), silicones, acrylic and methacrylic acid esters or polyvinyl alcohol-polyvinylacetate can be incorporated into the mixture without having to be physically changed by heat in order to be used as controlling agents. This implies a substantial difference compared to wax materials which need to be melted before being incorporated into the mixture.

One of the copolymers used in the present invention is Eudragit NE30D which is is the aqueous dispersion of a neutral copolymer based on ethyl acrylate and methyl methacrylate.

In another embodiment of the first aspect of the invention, the multiparticulate system comprises beads, spheroids, microspheres, seeds, pellets, granules, minitablets or microtablets, preferably granules or pellets.

In another embodiment of the first aspect of the invention, a hydrophobic agent and quetiapine hemifumarate are dispersed in a carrier matrix.

In another embodiment of the first aspect of the invention, the extended release multiparticulate system comprises inert cores coated with quetiapine hemifumarate and a hydrophobic release controlling agent.

In another embodiment of the first aspect of the invention, the extended release multiparticulate system contains at least one polyol selected from mannitol, xylitol, maltitol, lactitol, erythritol, isomalt, sorbitol and mixtures thereof.

In one embodiment of the third aspect of the invention, the granulate is prepared by single pot technology comprising at least the steps of:
a) Preparing a binder solution, optionally dissolving at least one hydrophobic release controlling agent in at least one solvent,
b) Incorporating the binder solution into the mixture to form a granulate,
c) Drying the granulate in the single pot at a temperature equal to or below 50 °C, optionally under vacuum.

In another embodiment of the third aspect of the invention, the granulate is prepared by fluid bed technology comprising at least the steps of:
a) Preparing the binder solution, optionally dissolving at least one hydrophobic release controlling agent in at least one solvent,
b) Spraying the binder solution onto the mixture to form a granulate,
c) Drying the granulate at inlet temperature equal or below 70 °C.

The extended release quetiapine formulations described in the examples can be prepared using the following equipment or equivalent: bin blenders, planetary mixers, turbula mixers, mixer granulators (Saizoner^{®}), extruders (Caleva model 15), oscillating granulators (VMG -8^{®}), high shear mixers (IKA^{®}), spheronizers (Caleva model 250), drum coating equipment (Neocota^{®}40T), fluid bed equipment (Glatt^{®} FBE 125) and ovens. The tablets can be obtained by a rotary (Killian^{®} LX-21) or an eccentric (Korsch^{®} EK II) tablet press. The multiparticulate system can be incorporated into capsules by a capsule machine like AF25T.

The extended release properties of the formulation of the present invention may be demonstrated by monitoring the dissolution of the active ingredient. The dissolution of the active ingredient may be monitored using standard procedures well known to those skilled in the art (e.g. the dissolution test procedures, such as the Rotating Basket Method (Apparatus I), disclosed in the U.S. Pharmacopeia (USP). In a particular example a tablet is immersed in about 900 ml of water with 0.05 M sodium citrate and 0.09 N sodium hydroxide (pH 4.7-4.9) and the dissolution profile is determined by the Rotating Basket method for 5 hours at a speed of 200 rpm. Aliquots of the medium are withdrawn at 1, 2, 4, and 5 hours. Then, adding 100 ml of 0.05 M sodium phosphate and 0.46 N sodium hydroxide to the dissolution media to afford a pH of 6.4 to 6.8 and withdrawing aliquots at 6, 12 and 20 hours.

The invention will be understood more clearly from the following non-limiting examples:

### EXAMPLES

### Example Q1 (sugar free tablets based on pellets with oily substances)

The following process was used to prepare 2500 tablets having the composition defined in Table 1. Quetiapine hemifumarate (1.133 kg), dicalcium phosphate (281.2 g), lactose (250 g), sorbitol (125 g), magnesium stearate (40 g) and talc (20 g) were blended in a mixer for approximately 3 minutes. The mixture was wet granulated in a mixer granulator using mineral oil (90.8 g) dissolved in acetone (250 ml). The wet mass was then gradually fed into a small scale extruder (1. 5 mm, 50 rpm) and the extrudate collected. The extrudate was spheronised at about 600 rpm for 5-10 minutes. The resulting pellets were transferred to a tray and dried in an oven at 50 °C overnight. These pellets were then mixed with a second portion of magnesium stearate (40 g) and talc (20 g). Finally, the pellets were compressed into tablets using a conventional rotary tablet press. Using conventional drum coating equipment the tablets were then sugar-free coated (7 % based on tablet weight) with an aqueous suspension (280 ml) of the sugar-free coating constituents (i.e. sorbitol (78.40 g), yellow ferric oxide (6.25 g), talc (42.00 g) and titanium dioxide (12.60 g)) at an inlet temperature of approximately 80 °C.

### Example Q2 (sugar free tablets based on granules obtained by fluid bed granulation)

The following process was used to prepare 2500 tablets having the composition defined in Table 1. A mixture of quetiapine hemifumarate (1.133 kg), dicalcium phosphate (281.2 g), lactose (250 g), sorbitol (125 g), magnesium stearate (40 g), talc (20 g) was placed in the top spraying chamber of a fluid bed granulator. A solution of mineral oil and acetone (90.80 g/250 ml) was sprayed onto this mixture with the following parameters: air flow 100-110 m³/h, liquid flow 6-7 g/min, inlet temperature 65 °C and spray pressure 2.2 bar. Once granulation was completed, the granules were passed through a sieve (1 mm mesh). These granules were then mixed with a second portion of magnesium stearate (40 g) and talc (20 g). Finally, the granules were compressed into tablets using a conventional rotary tablet press. Using conventional drum coating equipment the tablets were then sugar-free coated (7 % based on tablet weight) with an aqueous suspension (280 ml) of the sugar free coating constituents (i.e. sorbitol (78.40 g), yellow ferric oxide (6.25 g), talc (42.00 g) and titanium dioxide (12.60 g)) at an inlet temperature of approximately 80 °C.

### Example Q3 (sugar free tablets based on granules obtained by extrusion)

The following process was used to prepare 2500 tablets having the composition defined in Table 1. Quetiapine hemifumarate (1.133 kg), dicalcium phosphate (281.20 g), lactose (250 g), sorbitol (125 g), magnesium stearate (40 g) and talc (20 g) were blended in a mixer for approximately 3 minutes. The mixture was wet granulated in a mixer granulator using mineral oil (90.8 g) dissolved in acetone (250 ml). The wet mass was then gradually fed into a small scale extruder (1.5 mm, 50 rpm) and the extrudate collected. The resulting extrudate was granulated and transferred to a tray and dried in an oven at 50 °C overnight. This granulate was then mixed with a second portion of magnesium stearate (40 g) and talc (20 g). Finally, the granulate was compressed into tablets using a conventional rotary tablet press. Using conventional drum coating equipment the tablets were then sugar free coated (7 % based on tablet weight) with an aqueous suspension (280 ml) of the sugar free coating constituents (i.e. sorbitol (78.40 g), yellow ferric oxide (6.25 g), talc (42.00 g) and titanium dioxide (12.60 g)) at an inlet temperature of approximately 80 °C.

| **Table 1: Extended release quetiapine hemifumarate formulation with polyols (Examples Q1,Q2,Q3)** | | | |
|---|---|---|---|
| **Components** | **mg/tablet** | **Percentage (%)** | **Optimal percentage w/w (%)** |
| CORE | | | |
| Quetiapine hemifumarate (400 mg quetiapine) | 453.20 | 56.65 | 20.00-80.00 |
| Mineral oil | 36.32 | 4.54 | 1.00-5.00 |
| Sorbitol | 50.00 | 6.25 | 2.00-20.00 |
| Dicalcium phosphate | 112.48 | 14.06 | 10.00-18.75 |
| Lactose | 100.00 | 12.50 | 10.00-18.75 |
| Mg stearate | 32.00 | 4.00 | 0.25-5.00 |
| Talc | 16.00 | 2.00 | 1.00-10.00 |
| Acetone | qs | qs | qs |
| COATING | 56.00 | 7.00 | 2.00-15.00 |
| Sugar free suspension | | | |
| 50.00 % (w/v) | 31.36 | 28.00 | 10.00-30.00 |
| Sorbitol | 5.04 | 4.50 | 0.50-5.00 |
| Titanium dioxide | 2.80 | 2.50 | 0.01-3.00 |
| Colouring agent | 16.80 | 15.00 | 1.00-20.00 |
| Talc | qs | qs | qs |
| Water | | | |
| Total | 800.00 | 100.00 | 100.00 |

These examples were prepared replacing dicalcium phosphate by calcium sulphate or calcium carbonate and mineral oil by silicone oil.

### Example Q7 (sugar free tablets based on PLGA pellets extrusion/spheronization)

The following process was used to prepare 2500 tablets having the composition defined in Table 3. Quetiapine hemifumarate (1.133. kg), mannitol (367 g), sorbitol (140 g), magnesium stearate (40 g) and talc (20 g) were blended in a mixer for approximately 3 minutes. The mixture was wet granulated in a mixer granulator using PLGA (80 g) dissolved in acetone (250 ml). The wet mass was then gradually fed into a small scale extruder (1.5 mm, 50 rpm) and the extrudate collected. The extrudate was spheronised at about 600 rpm for 5-10 minutes. The resulting pellets were transferred to a tray and dried in an oven at 50 °C overnight. After that, these pellets were mixed with a second portion of magnesium stearate (40 g) and talc (20 g). Finally, the pellets were compressed into tablets using a conventional rotary tablet press. Using conventional drum coating equipment the tablets were then sugar free coated (7 % based on tablet weight) with an aqueous suspension (280 ml) of the sugar free coating constituents (i.e. sorbitol (78.40 g), yellow ferric oxide (6.25 g), talc (42.00 g) and titanium dioxide (12.60 g)) at an inlet temperature of approximately 80 °C.

### Example Q8 (sugar free tablets based on PLGA granules obtained by fluid bed granulation)

The following process was used to prepare 2500 tablets having the composition defined in Table 3. A mixture of quetiapine hemifumarate (1.133 kg), mannitol (367 g), sorbitol (140 g), magnesium stearate (40 g) and talc (20 g) were placed in the top spraying chamber of a fluid bed granulator. The solution of PLGA and acetone (100 g/250 ml) was sprayed onto this mixture with the following parameters: air flow 100-110 m³/h, liquid flow 6-7 g/min, inlet temperature 65 ° C, spray pressure 2.2 bar. Once granulation was complete, the granules were passed through a sieve (1 mm mesh). These granules were then mixed with the other portion of magnesium stearate (40 g) and talc (20 g). Finally, the granules were compressed into tablets using a conventional rotary tablet press. Using conventional drum coating equipment the tablets were then sugar free coated (7 % based on tablet weight) with an aqueous suspension (280 ml) of the sugar-free coating constituents (i.e. sorbitol (78.40 g), yellow ferric oxide (6.25 g), talc (42.00 g) and titanium dioxide (12.60 g)) at an inlet temperature of approximately 80 °C.

### Example Q9 (sugar free tablets based on PLGA granules obtained by extrusion)

The following process was used to prepare 2500 tablets having the composition defined in Table 3. Quetiapine hemifumarate (1.133 kg), mannitol (367 g), sorbitol (140 g), magnesium stearate (40 g) and talc (20 g) were blended in a mixer for approximately 3 minutes. The mixture was wet granulated in a mixer granulator using PLGA (100 g) dissolved in acetone (250 ml). The wet mass was then gradually fed into a small scale extruder (1.5 mm, 50 rpm) and the extrudate collected. The resulting extrudate was granulated and transferred to a tray and dried in an oven at 50 °C overnight. This granulate was then mixed with a second portion of magnesium stearate (40 g) and talc (20 g). Finally, the granulate was compressed into tablets using a conventional rotary tablet press. Using conventional drum coating equipment the tablets were then sugar free coated (7 % based on tablet weight) with a water suspension (280 ml) of the sugar free coating constituents (i.e. sorbitol (78.40 g), yellow ferric oxide (6.25 g), talc (42.00 g) and titanium dioxide (12.60 g)) at an inlet temperature of approximately 80 °C.

| **Table 3: Extended release quetiapine hemifumarate formulation based on PLGA and polyols (Examples Q7,Q8,Q9)** | | | |
|---|---|---|---|
| **Components** | **mg/tablet** | **Percentage (%)** | **Optimal percentage w/w (%)** |
| CORE | | | |
| Quetiapine hemifumarate (400 mg quetiapine) | 453.20 | 56.65 | 20.00-80.00 |
| Mannitol | 146.80 | 18.35 | 15.00-44.75 |
| Sorbitol | 56.00 | 7.00 | 2.00-20.00 |
| PLGA | 40.00 | 5.00 | 2.00-10.00 |
| Mg stearate | 32.00 | 4.00 | 0.25-5.00 |
| Talc | 16.00 | 2.00 | 1.00-10.00 |
| Acetone | qs | qs | qs |
| COATING | 56.00 | 7.00 | 2.00-15.00 |
| Sugar free suspension | | | |
| 50.00 % (w/v) | 31.36 | 28.00 | 10.00-30.00 |
| Sorbitol | 5.04 | 4.50 | 0.50-5.00 |
| Titanium dioxide | 2.80 | 2.50 | 0.01-3.00 |
| Colour | 16.80 | 15.00 | 1.00-20.00 |
| Talc | qs | qs | qs |
| Water | | | |
| Total | 800.00 | 100.00 | 100.00 |

### Example Q13 (inert cores and mineral oil coating)

The following process was used to prepare 2500 tablets having the composition defined in Table 5. Mannitol inert cores (Mcell^{®} 200) (525.20 g) were placed in the top spraying chamber of a fluid bed granulator. A solution of mineral oil (80 g), quetiapine hemifumarate (1.133 kg) and acetone (250 ml) was sprayed onto the inert cores with the following parameters: air flow 100-110 m³/h; liquid flow 6-7 g/min; inlet temperature 65 ° C and spray pressure 2.2 bar. Once coating was complete, the pellets were passed through a sieve (1 mm mesh). These pellets were then mixed with magnesium stearate (80 g) and talc (40 g). Finally, the pellets were compressed into tablets using a conventional rotary tablet press. Using conventional drum coating equipment the tablets were then sugar free coated (7 % based on tablet weight) with an aqueous suspension (280 ml) of the sugar free coating constituents (i.e. sorbitol (78.40 g), yellow ferric oxide (6.25 g), talc (42.00 g) and titanium dioxide (12.60 g)) at an inlet temperature of approximately 80 °C.

| **Table 5: Extended release quetiapine hemifumarate formulation based on inert cores (Example Q13)** | | | |
|---|---|---|---|
| **Components** | **mg/ tablet** | **Percentage (%)** | **Optimal percentage (%)** |
| CORES | | | |
| Quetiapine hemifumarate (400 mg quetiapine) | 453.20 | 56.65 | 20.00-80.00 |
| Mineral oil | 32.00 | 4.00 | 1.00-5.00 |
| Mannitol inert cores (Mcell® 200) | 210.08 | 26.35 | 20.00-37.75 |
| Mg stearate | 32.00 | 4.00 | 0.25-5.00 |
| Talc | 16.00 | 2.00 | 1.00-10.00 |
| Acetone | qs | Qs | qs |
| COATING | 56.00 | 7.00 | 2.00-15.00 |
| Sugar free solution 50.00 % (w/v) | | | |
| Sorbitol | 31.36 | 28.00 | 10.00-30.00 |
| Titanium dioxide | 5.04 | 4.50 | 0.50-5.00 |
| Colour | 2.80 | 2.50 | 0.00-3.00 |
| Talc | 16.80 | 15.00 | 1.00-20.00 |
| Water | qs | qs | Qs |
| Total | 800.00 | 100.00 | 100.00 |

### Example Q15 (dragees based on pellets obtained by extrusion/spheronization)

The following process was used to prepare tablets having the composition defined in Table 7. Quetiapine hemifumarate (1.133 kg), dicalcium phosphate (281.20 g), lactose (250 g), sucrose (125 g), magnesium stearate (40 g), talc (20 g) were blended in a mixer for approximately 3 minutes. The mixture was wet granulated in a mixer granulator using mineral oil (90.80 g) dissolved in acetone (250 ml). The wet mass was then gradually fed into a small scale extruder (1.5 mm, 50 rpm) and the extrudate collected. The extrudate was spheronised at about 600 rpm for 5-10 minutes. The resulting pellets were transferred to a tray and dried in an oven at 50 °C overnight. After that, these pellets were mixed with the other portion of magnesium stearate (40 g) and talc (20 g). Finally, the pellets were compressed into tablets using a conventional rotary tablet press. Using conventional drum coating equipment the tablets were then sugar coated (7 % based on tablet weight) with an aqueous suspension (233.33 ml) of the sugar coating constituents (i.e. sucrose (88.65 g), yellow ferric oxide (5.83 g), talc (35.00 g) and titanium dioxide (10.50 g)) at an inlet temperature of approximately 80 °C.

| **Table7: Extended release quetiapine hemifumarate formulation in dragees form** | | | | |
|---|---|---|---|---|
| **Components** | **Mg/tablet** | | | **Optimal percentage w/w (%)** |
| | **Example Q15,Q17,Q19** | **Example Q16** | **Example Q18** | |
| CORES | | | | |
| Quetiapine hemifumarate (400 mg quetiapine) | 453.20 | 453.20 | 453.20 | 20.00-80.00 |
| Mineral oil | 36.32 | --- | 70.00 | 1.00-10.00 |
| Dicalcium phosphate | 112.48 | 157.4 | 157.40 | 10.00-30.00 |
| Lactose | 100.00 | --- | --- | 10.00-18.875 |
| Sucrose | 50.00 | --- | 53.40 | 2.00-20.00 |
| Sorbitol | --- | 53.40 | --- | 2.00-20.00 |
| Mg stearate | 32.00 | 80.00 | 10.00 | 0.25-20.00 |
| Talc | 16.00 | --- | --- | 1.00-10.00 |
| Acetone | qs | --- | qs | --- |
| Water | --- | qs | --- | qs |
| COATING | 56.00 | 56.00 | 56.00 | 2.00-15.00 |
| Sucrose syrup 60 % (w/v) | | | | |
| Sucrose 38 % | 35.46 | 35.46 | 35.46 | 30.00-65.00 |
| Titanium dioxide 4.5 % | 4.20 | 4.20 | 4.20 | 0.50-5.00 |
| Colour 2.5 % | 2.33 | 2.33 | 2.33 | 0.01-5.00 |
| Talc 15 % | 14.00 | 14.00 | 14.00 | 0.00-20.00 |
| Water | qs | qs | qs | |
| Total | 800.00 | 800.00 | 800.00 | 100 |

### Example Q16 (dragees based on pellets obtained by extrusion/spheronization)

The following process was used to prepare 2500 tablets having the composition defined in Table 7. Quetiapine hemifumarate (1.133 kg), magnesium stearate (190 g) and dicalcium 5 phosphate (307.96 g) were blended in a mixer for approximately 3 minutes. The mixture was wet granulated in a mixer granulator using sorbitol (133.50 g) dissolved in water. The wet mass was then gradually fed into a small scale extruder (1.5 mm, 50 rpm) and the extrudate collected. The extrudate was spheronised at about 1000 rpm for 5-10 minutes. The resulting pellets were transferred to a tray and dried in an oven at 50 °C overnight. These pellets were then mixed with carbonate/ phosphate (100 g) and magnesium stearate (10 g). Finally, the pellets were compressed into tablets using a conventional rotary tablet press. Using conventional drum coating equipment the tablets were then sugar coated (7 % based on tablet weight) with an aqueous suspension (233.33 ml) of the sugar coating constituents (i.e. sucrose (88.65 g), yellow ferric oxide (5.83 g), talc (35.00 g) and titanium dioxide (10.50 g)) at an inlet temperature of approximately 80 °C.

### Example Q17 (dragees based on granules obtained by fluid bed granulation)

The following process was used to prepare 2500 tablets having the same composition as example Q15 defined in Table 7. A mixture of quetiapine hemifumarate (1.133 kg), dicalcium phosphate (281.20 g), lactose (250 g), sucrose (125 g), magnesium stearate (40 g), talc (20 g) were placed in the top spraying chamber of a fluid bed granulator. The solution of mineral oil (90.8 g) and acetone (250 ml) was sprayed onto this mixture with the following parameters: air flow 100-110 m³/h; liquid flow 6-7 g/min; inlet temperature 65 ° C; spraying pressure 2.2 bar. Once granulation was complete, the granules were passed through a sieve (1 mm mesh). After that, these granules were mixed with the second portion of magnesium stearate (40 g) and talc (20 g). Finally, the pellets were compressed into tablets using a conventional rotary tablet press. Using conventional drum coating equipment the tablets were then sugar coated (7 % based on tablet weight) with a water suspension (233.33 ml) of the sugar coating constituents (i.e. sucrose (88.65 g), yellow ferric oxide (5.83 g), talc (35.00 g) and titanium dioxide (10.50 g)) at an inlet temperature of approximately 80 °C.

### Example Q18 (dragees based on granules obtained by fluid bed granulation)

The following process was used to prepare 2500 tablets having the composition defined in Table 7. A mixture of quetiapine hemifumarate (1 kg), dicalcium phosphate (307.96 g), magnesium stearate (20 g) and sucrose (133.50 g) were placed in the top spraying chamber of a fluid bed granulator. The solution of mineral oil (175 g) and acetone was sprayed onto this mixture with the following parameters: air flow (m³/h) 100-110 m³/h; liquid flow (g/min) 6-7 g/min, inlet temperature 65 °C and spray pressure 2.2 bar. Once granulation was complete, the granules were passed through a sieve (1 mm mesh). These granules were then mixed with dicalcium phosphate (100 g) and magnesium stearate (5 g). Finally, the granules were compressed into tablets using a conventional rotary tablet press. Using conventional drum coating equipment the tablets were then sugar coated (7 % based on tablet weight) with an aqueous suspension (233.33 ml) of the sugar-coating constituents (i.e. sucrose (88.65 g), yellow ferric oxide (5.83 g), talc (35.00 g) and titanium dioxide (10.50 g)) at an inlet temperature of approximately 80 °C.

### Example Q19 (dragees based on granules obtained by extrusion)

The following process was used to prepare 2500 tablets having the same composition as example Q15 defined in Table 7. Quetiapine hemifumarate (1.133 kg), dicalcium phosphate (281.20 g), lactose (250 g), sucrose (125 g), magnesium stearate (40 g), talc (20 g) were blended in a mixer for approximately 3 minutes. The mixture was wet granulated in a mixer granulator using mineral oil (90.80 g) dissolved in acetone (250 ml). The wet mass was then gradually fed into a small scale extruder (1.5 mm, 50 rpm) and the extrudate collected. The resulting extrudate was granulated andtransferred to a tray and dried in an oven at 50 °C overnight. After that, this granulate was mixed with a second portion of magnesium stearate (40 g) and talc (20 g). Finally, granulate was compressed into tablets using a conventional rotary tablet press. Using conventional drum coating equipment the tablets were then sugar coated (7 % based on tablet weight) with an aqueous suspension (233.33 ml) of the sugar coating constituents (i.e. sucrose (88.65 g), yellow ferric oxide (5.83 g), talc (35.00 g) and titanium dioxide (10.50 g)) at an inlet temperature of approximately 80 °C.

All the above described formulations were also prepared by sealing the tablets with a coating well known in the art, before the sugar or sugar free coating was applied. A 20 % (w/v) ethanolic shellac solution was sprayed onto the cores using the minimum quantity of coating that would not affect drug release (approximately between 1 to 3 %).

### Example Q25 (tablets based on granules with Eudragit NE30D obtained by fluid bed granulation)

The following process was used to prepare 2500 tablets having the composition defined in Table 9. A mixture of quetiapine hemifumarate (1.133 kg), dicalcium phosphate (280 g), lactose (267 g), magnesium stearate (40 g) and talc (20 g) were placed in the top spraying chamber of a fluid bed granulator. The dispersion of Eudragit NE 30D (666.66 ml) was sprayed onto the mixture with the following parameters: air flow 100-110 m³/h, liquid flow 6-7 g/min, inlet temperature 65 °C and spray pressure 2.2 bar. Once the granulation was complete, the granules were passed through a sieve (1 mm mesh). These granules were then mixed with a second portion of magnesium stearate (40 g) and talc (20 g). Finally, the granules were compressed into tablets using a conventional rotary tablet press. Using conventional drum coating equipment the tablets were then film coated with an aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

### Example Q26 (tablets based on granules with Eudragit NE30D in a mixer granulator)

The following process was used to prepare 2500 tablets having the same composition as example Q25 defined in Table 9. Quetiapine hemifumarate (1.133 kg), dicalcium phosphate (280 g), lactose (267 g), magnesium stearate (40 g) and talc (20 g) were placed in a mixer granulator. The dispersion of Eudragit NE 30D (666.66 ml) was added while mixing until a suitable mass was obtained. The wet granules were passed through a sieve (1 mm mesh) and dried in an oven at 50 °C overnight. These granules were then mixed with a second portion of magnesium stearate (40 g) and talc (20 g). Finally, the granules were compressed into tablets using a conventional rotary tablet press. Using conventional drum coating equipment the tablets were then film coated with 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

### Example Q27 (tablets based on granules with Eudragit NE30D and oil coating)

The following process was used to prepare 2500 tablets having the composition defined in Table 9. Quetiapine hemifumarate (1.133 kg), dicalcium phosphate (280 g), lactose (267 g), magnesium stearate (40 g) and talc (20 g) were placed in a mixer granulator. The dispersion of Eudragit NE 30D (666.66 ml) was added while mixing until a suitable mass was obtained. The wet granules were passed through a sieve (1 mm mesh) and dried in an oven at 50 °C overnight. These granules were then placed in the top spraying chamber of a fluid bed granulator. A solution of hydrogenated castor oil in acetone (20 %) was sprayed onto the active ingredient with the following parameters: air flow (m³/h) 100-110 m³/h, liquid flow (g/min) 6-7 g/min, inlet temperature 65 °C and spray pressure 2.2 bar. These granules were then mixed with a second portion of magnesium stearate (40 g) and talc (20 g). Finally, the granules were compressed into tablets using a conventional rotary tablet press. Using conventional drum coating equipment the tablets were then film coated with a 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

| **Table 9: Extended release quetiapine hemifumarate formulations based on copolymer of acrylic and methacrylic acid esters** | | | |
|---|---|---|---|
| | **mg/tablet** | | **Optimal percentage w/w(%)** |
| **Components** | **Example Q25, Q26** | **Example Q27** | |
| CORES | | | |
| Quetiapine hemifumarate (400 mg quetiapine) | 453.20 | 453.20 | 20.00-80.00 |
| Dicalcium phosphate | 104.00 | 136.00 | 0.00-25.00 |
| Lactose | 106.80 | 106.80 | 10.00-25.00 |
| Eudragit NE 30D (30% | | | |
| polymer content) | 80.00 | 56.00 | 5.00-20.00 |
| Magnesium stearate | 32.00 | 32.00 | 0.25-5.00 |
| Talc | 16.00 | 16.00 | 1.00-10.00 |
| COATING | 8.00 | 25.00 | 2.00-15.00 |
| Hydrogenated castor oil | --- | 20.00 | 0.00-25.00 |
| Hydroxypropyl | | | |
| methylcellulose | 5.00 | 5.00 | 1.00-10.00 |
| Polyethylene glycol 400 | 1.00 | 1.00 | 0.05-2.00 |
| Yellow ferric oxide | 0.15 | 0.15 | 0.05-0.10 |
| Titanium dioxide | 1.85 | 1.85 | 0.10-1.00 |
| Total | 800.00 | 800.00 | 100 |

### Example Q28 (tablets based on granules with Eudragit NE30D by fluid bed granulation)

The following process was used to prepare 2500 tablets having the defined in Table 10. Quetiapine fumarate (1 kg), dicalcium phosphate (280 g), lactose (400 g), magnesium stearate (40 g) and talc (20 g) were placed in the top spraying chamber of a fluid bed granulator. The dispersion of Eudragit NE 30D (666.66 ml) was sprayed onto the active ingredient with the following parameters: air flow 100-110 m³/h, liquid flow 6-7 g/min, inlet temperature 65 ° C and spraying pressure 2.2 bar. Once granulation is complete, the granules are passed through a sieve (1 mm mesh). After that, these granules were mixed with a second portion of magnesium stearate (40 g) and talc (20 g). Finally, granules were compressed into tablets using a conventional rotary tablet press. The tablets were then film coated using conventional drum coating equipment with an aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

### Example Q29 (tablets based on granules with Eudragit NE30D)

The following process was used to prepare 2500 tablets having the same composition than example Q28 defined in Table 10. Quetiapine fumarate (1 kg), dicalcium phosphate (280 g), lactose (400 g), magnesium stearate (40 g) and talc (20 g) were placed in a mixer granulator. The dispersion of Eudragit NE 30D (666.66 ml) was added while mixing until a suitable mass was obtained. The wet granules were passed through a sieve (1 mm mesh) and dried in an oven at 50 °C overnight. After that, these granules were mixed with a second portion of magnesium stearate (40 g) and talc (20 g). Finally, granules were compressed into tablets using a conventional rotary tablet press. The tablets were then film coated using conventional drum coating equipment with 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

### Example Q30 (tablets based on granules with Eudragit NE30D and oil coating)

The following process was used to prepare 2500 tablets having the composition defined in Table 10. Quetiapine fumarate (1 kg), dicalcium phosphate (280 g), lactose (400 g), magnesium stearate (40 g) and talc (20 g) were placed in a mixer granulator. The dispersion of Eudragit NE 30D (666.66 ml) was added while mixing until a suitable mass was obtained. The wet granules were passed through a sieve (1 mm mesh) and dried in an oven at 50 °C overnight. After that, these granules were placed in the top spraying chamber of a fluid bed granulator. A solution of hydrogenated castor oil in acetone (20 %) was sprayed onto the active ingredient with the following parameters: air flow (m³/h) 100-110 m³/h, liquid flow (g/min) 6-7 g/min, inlet temperature 65 ° C and spraying pressure 2.2 bar. After that, these granules were mixed with a second portion of magnesium stearate (40 g) and talc (20 g). Finally, granules were compressed into tablets using a conventional rotary tablet press. The tablets were then film coated using conventional drum coating equipment with 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

| **Table 10: Extended release quetiapine fumarate formulations based on copolymer of acrylic and methacrylic acid esters** | | | |
|---|---|---|---|
| | **mg/tablet** | | **Optimal percentage w/w(%)** |
| **Components** | **Example Q28, Q29** | **Example Q30** | |
| CORES | | | |
| Quetiapine fumarate | 400.00 | 400.00 | 20.00-80.00 |
| Dicalcium phosphate | 104.00 | 136.00 | 0.00-25.00 |
| Lactose | 160.00 | 160.00 | 10.00-25.00 |
| Eudragit NE 30D (30% | | | |
| polymer content) | 80.00 | 56.00 | 5.00-20.00 |
| Magnesium stearate | 32.00 | 32.00 | 0.25-5.00 |
| Talc | 16.00 | 16.00 | 1.00-10.00 |
| COATING | 8.00 | 25.00 | 2.00-15.00 |
| Hydrogenated castor oil | --- | 20.00 | 0.00-25.00 |
| Hydroxypropyl | | | |
| methylcellulose | 5.00 | 5.00 | 1.00-10.00 |
| Polyethylene glycol 400 | 1.00 | 1.00 | 0.05-2.00 |
| Yellow ferric oxide | 0.15 | 0.15 | 0.05-0.10 |
| Titanium dioxide | 1.85 | 1.85 | 0.10-1.00 |
| Total | 800.00 | 800 | 100 |

### Example Q31 (tablets based on granules with polyvinyl acetate)

The following process was used to prepare 2500 tablets having the composition defined in Table 11. A mixture of quetiapine hemifumarate (1.133 kg) and lactose monohydrate (610.50 g) were placed in the top spraying chamber of a fluid bed granulator. The polyvinyl acetate dispersion (Kollicoat^{®} SR 30 D) (666.66 ml) was sprayed onto the previous mixture with the following parameters: air flow 100-110 m³/h; liquid flow10 g/min, inlet temperature 55 °C and spray pressure 2.2 bar. Once granulation was complete, the granules were passed together with magnesium stearate (10 g) and colloidal silica (Aerosil^{®} 200) (10 g) through a sieve (1 mm mesh) and blended for 10 in a mixer. The mixture was then compressed using a conventional rotary tablet press. Using conventional drum coating equipment the tablets were then film coated with a 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

### Example Q32 (tablets based on granules with polyvinyl acetate and mineral oil)

The following process was used to prepare 2500 tablets having the composition defined in Table 11. Quetiapine hemifumarate (1 kg) and lactose monohydrate (591.03 g) were placed in a mixer granulator. The polyvinyl acetate dispersion (Kollicoat^{®} SR 30 D) (666.66 ml) and mineral oil (20 g) were added while mixing until a suitable mass was obtained. Once granulation was complete, the granules were passed together with magnesium stearate (10 g) and colloidal silica (Aerosil^{®} 200) (10 g) through a sieve (1 mm mesh) and blended for 10 min in a mixer. The mixture was the compressed using a conventional rotary tablet press. Using conventional drum coating equipment the tablets were then film coated with a 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

| **Table 11 : Extended release quetiapine hemifumarate formulations based on polyvinyl acetate** | | | |
|---|---|---|---|
| | **mg/tablet** | | **Optimal percentage w/w (%)** |
| | **Example Q31** | **Example Q32** | |
| CORES | | | |
| Quetiapine hemifumarate (400 mg quetiapine) | 453.20 | 453.20 | 20.00-80.00 |
| Lactose monohydrate | 250.80 | 242.80 | 10.00-40.00 |
| Polyvinyl acetate | | | |
| (Kollicoat® SR 30 D) | 80.00 | 80.00 | 5.00-20.00 |
| Mineral oil | --- | 8.00 | 0.00-2.00 |
| Magnesium stearate | 4.00 | 4.00 | 0.25-5.00 |
| Colloidal silica | 4.00 | 4.00 | 1.00-10.00 |
| COATING | 8.00 | 8.00 | 2.00-15.00 |
| Hydroxypropyl | | | |
| methylcellulose | 5.00 | 5.00 | 1.00-10.00 |
| Polyethylene glycol 400 | 1.00 | 1.00 | 0.05-2.00 |
| Yellow ferric oxide | 0.15 | 0.15 | 0.05-0.10 |
| Titanium dioxide | 1.85 | 1.85 | 0.10-1.00 |
| Total | 800.00 | 800.00 | 100 |

### Example Q33 (tablets based on granules with polyvinyl acetate)

The following process was used to prepare 2500 tablets having the composition defined in Table 12. Quetiapine fumarate (1 kg) and lactose monohydrate (740 g) were placed in the top spraying chamber of a fluid bed granulator. The acetate polyvinyl dispersion (Kollicoat^{®} SR 30 D) (666.66 ml) was sprayed onto the previous mixture with the following parameters: air flow 100-110 m³/h; liquid flow10 g/min, inlet temperature 55 °C and spraying pressure 2.2 bar. Once granulation is complete, the granules are passed together with magnesium stearate (10 g) and colloidal silica (Aerosil^{®} 200) (10 g) through a sieve (1 mm mesh) and blended for 10 in a mixer. After that, the mixture was tableted using a conventional rotary tablet press. The tablets were then film coated using conventional drum coating equipment with 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

### Example Q34 (tablets based on granules with polyvinyl acetate and mineral oil)

The following process was used to prepare 2500 tablets having the composition defined in Table 12. Quetiapine fumarate (1 kg) and lactose monohydrate (760 g) were placed in a mixer granulator. The acetate polyvinyl dispersion (Kollicoat^{®} SR 30 D) (666.66 ml) and mineral oil (20 g) was added while mixing until a suitable mass was obtained. Once granulation is complete, the granules are passed together with magnesium stearate (10 g) and colloidal silica (Aerosil^{®} 200) (10 g) through a sieve (1 mm mesh) and blended for 10 in a mixer. After that, the mixture was tableted using a conventional rotary tablet press. The tablets were then film coated using conventional drum coating equipment with 100 ml aqueous suspension of the film coating constituents (i.e. hydroxypropyl methylcellulose (12.5 g), polyethylene glycol 400 (2.5 g), yellow ferric oxide (0.375 g) and titanium dioxide (4.625 g)) at an inlet temperature of approximately 80 °C.

| **Table 12: Extended release quetiapine fumarate formulations based on polyvinyl acetate** | | | |
|---|---|---|---|
| | **mg/tablet** | | **Optimal percentage w/w(%)** |
| | **Example Q33** | **Example Q34** | |
| CORES | | | |
| Quetiapine fumarate | 400.00 | 400.00 | 20.00-80.00 |
| Lactose monohydrate | 304.00 | 296.00 | 10.00-25.00 |
| Polyvinyl acetate (Kollicoat® SR 30 D) | 80.00 | 80.00 | 5.00-20.00 |
| Mineral oil | --- | 8.00 | 0.00-2.00 |
| Magnesium stearate | 4.00 | 4.00 | 0.25-5.00 |
| Colloidal silica | 4.00 | 4.00 | 1.00-10.00 |
| COATING | 8.00 | 8.00 | 2.00-15.00 |
| Hydroxypropyl | | | |
| methylcellulose | 5.00 | 5.00 | 1.00-10.00 |
| Polyethylene glycol 400 | 1.00 | 1.00 | 0.05-2.00 |
| Yellow ferric oxide | 0.15 | 0.15 | 0.05-0.10 |
| Titanium dioxide | 1.85 | 1.85 | 0.10-1.00 |
| Total | 800.00 | 800.00 | 100 |

### EXAMPLE Q35 DISSOLUTION TEST METHOD

Equipment: Apparatus 2 (paddle apparatus); temperature: 37 °C±0.5 °C; Medium:
Phosphate buffer pH 6.8; Volume: 900 ml; Speed: 100 rpm

For preparing 25 I of medium 170.1 g potassium dihydrogenphosphate and 22.4 g sodium hydroxide are solved in 1000.0 ml purified water. Water is added to complete to 25 I. If necessary pH is adjusted to 6.8 ± 0.05.

### Example Q36 (tablets based on granules with silicone oil)

**Table 13**

| Component | Amount/batch (g) |
|---|---|
| Quetiapine fumarate | 113,300 |
| Silicone oil 100 | 9,075 |
| Sorbitol | 12,500 |
| Dicalcium phosphate | 28,125 |
| Lactose | 25,000 |
| Talc | 4,000 |
| Magnesium stearate | 8,000 |

The following process was used to prepare 500 tablets of 200 mg Quetiapine having the composition defined in Table 13. Quetiapine fumarate (113.3 g), lactose (25 g), Dicalcium phosphate (28.125 g) and sorbitol (12.5 g) were placed in a mixer granulator. Silicone oil 100 (9.075 g) and water (25 g) were added while mixing until a suitable mass was obtained. Once granulation is complete, the granules were dried and passed through a sieve of 0.8 mm. The dried granules were blended with magnesium stearate (8 g) and Talc (4 g). After that, the mixture was tableted using a conventional rotary tablet press.

The dissolution results according to the method included in example Q35 are as follows: After 1 hour: 35 %; after 2 hours: 77 %; after 3 hours: 92 %.

### Example Q37 (tablets based on granules with mineral oil)

**Table 14**

| Component | Amount/batch (g) |
|---|---|
| Quetiapine fumarate | 113,300 |
| Mineral oil | 4,540 |
| Soybean lecithin | 4,540 |
| Sorbitol | 12,500 |
| Dicalcium phosphate | 28,125 |
| Lactose | 25,000 |
| Talc | 4,000 |
| Magnesium stearate | 8,000 |

The following process was used to prepare 500 tablets of 200 mg Quetiapine having the composition defined in Table 14. Quetiapine fumarate (113.3 g), lactose (25 g), Dicalcium phosphate (28.125 g) and sorbitol (12.5 g) were placed in a mixer granulator. Mineral oil (4.540 g), Soybean lecithin (4.540 g) and Water (25 g) were added while mixing until a suitable mass was obtained. Once granulation is complete, the granules were dried and passed through a sieve of 0.8 mm. The dried granules were blended with magnesium stearate (8 g) and Talc (4 g). After that, the mixture was tableted using a conventional rotary tablet press.

The dissolution results according to the method included in example Q35 are as follows: After 1 hour: 25 %; after 2 h: 49 %; after 4 h: 70 %; after 6 h: 75 %; after 16 h: 92 %

### Example Q38 (tablets based on granules with magnesium stearate)

**Table 15**

| Component | Amount/batch (g) |
|---|---|
| Quetiapine fumarate | 113,300 |
| Isomalt | 36,500 |
| Povidone | 2.100 |
| Lactose | 32,000 |
| Talc | 3.500 |
| Magnesium stearate | 22,600 |

The following process was used to prepare 500 tablets of 200 mg Quetiapine having the composition defined in Table 15. Quetiapine fumarate (113.3 g), Lactose (32 g), Isomalt (36.500 g) and Magnesium stearate (22.6 g) were placed in a mixer granulator. Povidone (2.1 g) was solved in Water (30 g) and added while mixing until a suitable mass was obtained. Once granulation is complete, the granules were dried and passed through a sieve of 0.8 mm. The dried granules were blended with Talc (3.5 g). After that, the mixture was tableted using a conventional rotary tablet press.

The dissolution results according to the method included in example Q35 are as follows: After 1 hour: 19 %; after 2 hours: 39 %; after 4 hours: 77 %; after 12 hours: 91 %.

### Example Q39 (pellets based on copolymer of acrylic and methacrylic acid esters

**Table 16**

| Component | Amount/batch (g) |
|---|---|
| Quetiapine fumarate | 230.240 |
| Microcrystalline cellulose | 54.960 |
| Povidone | 20.800 |
| Dicalcium phosphate | 100.000 |
| Eudragit RS PO | 100.000 |

The following process was used to prepare 506 g pellets having the composition defined in Table 16. Quetiapine fumarate (230.240 g), Microcrystalline cellulose (54.96 g), Dicalcium Phosphate (100 g) and Eudragit RS PO (100 g) were placed in a mixer granulator. Povidone (20.8 g) was solved in Water (140 g) and added while mixing until a suitable mass was obtained. Once granulation is completed, the mass is passed by a extruder provided with a screen of 0.6 mm. The extruded material is spheronized in a spheronizer and then dried and calibrated.

The dissolution results according to the method included in example Q35 are as follows: After 4 hours: 61 %; after 7 hours: 71 %; after 10 hours: 77 %; after 20 hours: 90 %.

**Table 17**

| Component | Amount/batch (g) |
|---|---|
| Quetiapine fumarate | 230.240 |
| Microcrystalline cellulose | 54.960 |
| Povidone | 20.800 |
| Dicalcium phosphate | 100.000 |
| Eudragit RS 30D | *140.000 |
| Eudragit RS PO | 58.000 |

| | |
|---|---|
| * Equivalent to 42.00 g dry substance. | |

The following process was used to prepare 506 g pellets having the composition defined in Table 17. Quetiapine fumarate (230.240 g), Microcrystalline cellulose (54.96 g), Dicalcium Phosphate (100 g) and Eudragit RS PO (58 g) were placed in a mixer granulator. Povidone (20.8 g) was solved in Eudragit RS 30D (140 g) and added while mixing until a suitable mass was obtained. Once granulation is completed, the mass is passed by a extruder provided with a screen of 0.6 mm. The extruded material is spheronized in a spheronizer and then dried and calibrated. Eudragit^{®} RS PO is a powder and Eudragit^{®} RS 30 D a dispersion (30 % in water) of Meth-/acrylate copolymers with trimethyl-ammonioethylmethacrylate as a functional group.

The dissolution results according to the method included in example Q35 are as follows: After 4 hours: 56 %; after 7 hours: 68 %; after 10 hours: 76 %; after 20 hours: 89 %.

**Table 18**

| Component | Amount/batch (g) |
|---|---|
| Quetiapine fumarate | 230.240 |
| Microcrystalline cellulose | 51.760 |
| Povidone | 12.000 |
| Dicalcium phosphate | 50.000 |
| Eudragit RL 30D | *120.000 |
| Eudragit RL PO | 60.000 |

| | |
|---|---|
| * Equivalent to 36.00 g dry substance. | |

The following process was used to prepare 440 g pellets having the composition defined in Table 18. Quetiapine fumarate (230.240 g), Microcrystalline cellulose (51.76 g), Dicalcium Phosphate (50 g) and Eudragit RL PO (60 g) were placed in a mixer granulator. Povidone (12.0 g) was solved in Eudragit RL 30D (120 g) and added while mixing until a suitable mass was obtained. Once granulation is completed, the mass is passed by a extruder provided with a screen of 0.6 mm. The extruded material is spheronized in a spheronizer and then dried and calibrated.

Eudragit^{®} RL PO is a powder and Eudragit^{®} RL 30 D a dispersion (30 % in water) of Meth/acrylate copolymers with trimethyl-ammonioethylmethacrylate as a functional group.

The dissolution results according to the method included in example Q35 are as follows: After 4 hours: 61 %; after 7 hours: 76 %; after 10 hours: 86 %; after 20 hours: 100 %.

**Table 19**

| Component | Amount/batch (g) |
|---|---|
| Quetiapine fumarate | 230.240 |
| Microcrystalline cellulose | 51.760 |
| Povidone | 12.000 |
| Dicalcium phosphate | 50.000 |
| Eudragit FS 30D | *120.000 |

| | |
|---|---|
| * Equivalent to 36.00 g dry substance. | |

The following process was used to prepare 380 g pellets having the composition defined in Table 19. Quetiapine fumarate (230.240 g), Microcrystalline cellulose (51.76 g) and Dicalcium Phosphate (50 g) were placed in a mixer granulator. Povidone (12.0 g) was solved in Eudragit FS 30D (120 g) and added while mixing until a suitable mass was obtained. Once granulation is completed, the mass is passed by a extruder provided with a screen of 0.6 mm. The extruded material is spheronized in a spheronizer and then dried and calibrated.

Eudragit^{®} FS 30 D is a dispersion (30 % in water) of an anionic polymer with methacrilic acid as a functional group.

### Example Q40 (pellets based on polyvinyl acetate

**Table 20**

| Component | Amount/batch (g) |
|---|---|
| Quetiapine fumarate | 230.240 |
| Microcrystalline cellulose | 51.760 |
| Povidone | 12.000 |
| Dicalcium phosphate | 50.000 |
| Kollicoat SR 30D | *120.000 |

| | |
|---|---|
| * Equivalent to 36.00 g dry substance. | |

The following process was used to prepare 380 g pellets having the composition defined in Table 20. Quetiapine fumarate (230.240 g), Microcrystalline cellulose (51.76 g), Dicalcium Phosphate (50 g) were placed in a mixer granulator. Povidone (12.0 g) was solved in Kollicoat RL 30D (120 g) and added while mixing until a suitable mass was obtained. Once granulation is completed, the mass is passed by a extruder provided with a screen of 0.6 mm. The extruded material is spheronized in a spheronizer and then dried and calibrated.

Kollicoat^{®} SR 30 D consists in a dispersion in water of about 27 % polyvinyl acetate, 2.7 % povidone and 0.3 % sodium lauryl sulphate. It meets the requirement of the current monography for Poly (Vinyl Acetate) Dispersion 30 % of Ph. Eur.

**Table 21**

| Component | Amount/batch (g) |
|---|---|
| Quetiapine fumarate | 230.240 |
| Microcrystalline cellulose | 51.760 |
| Povidone | 12.000 |
| Dicalcium phosphate | 50.000 |
| Polyvinyl Acetate | 36.000 |
| Kollicoat SR 30D | *120.000 |

| | |
|---|---|
| * Equivalent to 36.00 g dry substance. | |

The following process was used to prepare 380 g pellets having the composition defined in Table 21. Quetiapine fumarate (230.240 g), Microcrystalline cellulose (51.76 g), Dicalcium Phosphate (50 g) and Polyvinyl acetate (36 g) were placed in a mixer granulator. Povidone (12.0 g) was solved in Kollicoat SR 30D (120 g) and added while mixing until a suitable mass was obtained. Once granulation is completed, the mass is passed by a extruder provided with a screen of 0.6 mm. The extruded material is spheronized in a spheronizer and then dried and calibrated.

### Example Q41 (pellets based on polyvinyl acetate and on copolymer of acrylic and methacrylic acid esters)

**Table 22**

| Component | Amount/batch (g) |
|---|---|
| Quetiapine fumarate | 230.240 |
| Microcrystalline cellulose | 51.760 |
| Povidone | 12.000 |
| Dicalcium phosphate | 50.000 |
| Eudragit RS PO | 58.000 |
| Kollicoat SR 30D | *120.000 |

| | |
|---|---|
| * Equivalent to 36.00 g dry substance. | |

The following process was used to prepare 438 g pellets having the composition defined in Table 22. Quetiapine fumarate (230.240 g), Microcrystalline cellulose (51.76 g), Dicalcium Phosphate (50 g) and Eudragit RS PO (58 g) were placed in a mixer granulator. Povidone (12.0 g) was solved in Kollicoat RL 30D (120 g) and added while mixing until a suitable mass was obtained. Once granulation is completed, the mass is passed by a extruder provided with a screen of 0.6 mm. The extruded material is spheronized in a spheronizer and then dried and calibrated.

### Comparative example 1 (pellets without delaying polymer)

This example does not form part of the invention. The example serves to check that microcrystalline cellulose, considered as a gelling agent in EP907364 A1, is not useful to achieve an extended release of quetiapine, and that it is the hydrophobic agent the one responsible for the extended release profile.

**Table 23**

| Component | Amount/batch (g) |
|---|---|
| Quetiapine fumarate | 230.240 |
| Microcrystalline cellulose | 100.000 |
| Povidone | 20.800 |
| Dicalcium phosphate | 129.600 |

The following process was used to prepare 480.64 g pellets having the composition defined in Table 23. Quetiapine fumarate (230.240 g), Microcrystalline cellulose (100.00 g) and Dicalcium Phosphate (129.60 g) were placed in a mixer granulator. Povidone (20.8 g) was solved in Water (180 g) and added while mixing until a suitable mass was obtained. Once granulation is completed, the mass is passed by a extruder provided with a screen of 0.6 mm. The extruded material is spheronized in a spheronizer and then dried and calibrated.

The dissolution results according to the method included in example Q35 are as follows: After 4 hours: 81 %; after 7 hours: 94 %; after 10 hours: 99 %.

## Claims

1. An extended release multiparticulate system comprising quetiapine or a pharmaceutical acceptable salt, pharmaceutical acceptable excipients and at least one hydrophobic release controlling agent, wherein the hydrophobic release controlling agent ranges from 1 % to 25 % w/w, preferably from 5 % to 20 %, more preferably from 5% to 10%.

2. The extended release system according to claim 1, comprising at least one hydrophobic release controlling agent selected from oils, fatty acids, salts of fatty acids, copolymers and polyvinyl acetate and mixtures thereof.

3. The extended release system according to claim 2, comprising at least one copolymer selected from siloxane, acrylic and methacrylic acid esters or polyvinyl alcohol-polyvinyl acetate and mixtures thereof.

4. The extended release system according to any one of the preceding claims, wherein the multiparticulate system are beads, spheroids, microspheres, seeds, pellets, granules, minitablets or microtablets, preferably granules or pellets.

5. The extended release system according to any one of the preceding claims, wherein a hydrophobic agent and quetiapine hemifumarate are dispersed in a carrier matrix.

6. The extended release system according to any one of the claims 1 to 5, comprising inert cores coated with quetiapine hemifumarate and a hydrophobic release controlling agent.

7. The extended release system according to any one of the preceding claims, wherein the inert cores comprise at least one polyol selected from mannitol, xylitol, maltitol, lactitol, erythritol, isomalt, sorbitol and mixtures thereof.

8. A tablet or capsule comprising the extended release system according to any of the preceding claims.

9. A method for the manufacture of a tablet according to claim 8, comprising at least the steps of:
a) Granulating a mixture of quetiapine hemifumarate and at least one pharmaceutical acceptable excipient with at least one hydrophobic release controlling agent,
b) Optionally, spheronizing the mixture to form pellets,
c) Drying the granulate or pellets,
d) Optionally, coating the dried product with at least one hydrophobic release controlling agent,
e) If needed, mixing the product obtained in the granulation or spheronization step with pharmaceutical acceptable excipients to obtain a compression mixture,
f) Compressing the previous mixture to obtain tablets, and optionally, applying a coating.

10. A method for the manufacture of a tablet according to claim 8, comprising at least the steps of: a) Providing the active substance, and optionally, mixing it with at least one pharmaceutically acceptable excipient, and or with at least one hydrophobic release controlling agent until a homogenous mixture is formed, b) Subjecting the active substance or the mixture to compression, c) Milling, grinding or sieving the compressed mixture, d) Optionally, adding additional excipients and mixing the combination, e) Compressing the pre-compressed product to form a tablet, and optionally coating.

11. A method for the manufacture of a tablet according to claim 8, comprising at least the steps of:
a) Providing the active substance, and optionally, mixing it with at least one pharmaceutical acceptable excipient and or with at least one hydrophobic release controlling agent until a homogenous mixture is formed,
b) Subjecting the active substance or the mixture to compression to form a tablet,
c) Optionally, coating.

12. A method for the manufacture of a tablet according to claim 8, comprising at least the steps of:
a) Coating inert cores with a coating organic solution which contains quetiapine hemifumarate and a hydrophobic release controlling agent,
b) If needed, mixing the coated cores with further pharmaceutical acceptable excipients to obtain a compression mixture,
c) Compressing the coated cores or the previous mixture to obtain tablets, and optionally, applying a coating.
